# EUROPEAN PATENT SPECIFICATION

(11) **EP 2 127 644 B1**
(45) Date of publication and mention of the grant of the patent: **23.02.2011**
(21) Application number: 09251404.1
(22) Date of filing: 27.05.2009
(51) Int. Cl.: A61K 9/70, C08L 13/00

(54) **Gel composition for medical material or hygiene material, molded article thereof and adhesive material or adhesive preparation using same**
Gelzusammensetzung für medizinisches Material oder Hygienematerial, Formteil davon und Klebstoffmaterial oder Klebstoffpräparat, das diese Zusammensetzung einsetzt
Composition de gel pour matériel médical ou matériel d'hygiène, son article moulé et matériel adhésif ou préparation adhésive l'utilisant

(30) Priority: 27.05.2008 JP 2008138733
(43) Date of publication of application: 02.12.2009
(73) Proprietor: Nitto Denko Corporation, Ibaraki-shi Osaka 567-8680 (JP)
(72) Inventor: Funakoshi, Yoshio, Ibaraki-shi Osaka 567-8680 (JP); Hamada, Atsushi, Ibaraki-shi Osaka 567-8680 (JP); Ishikura, Jun, Ibaraki-shi Osaka 567-8680 (JP); Kasahara, Tsuyoshi, Ibaraki-shi Osaka 567-8680 (JP)
(74) Representative: Duckworth, Timothy John

(56) References cited:
- EP-A- 2 002 824
- JP-A- 10 151 185

## Description

### Technical Field

The present invention relates to a gel composition suitable as a medical material or a hygiene material, and a molded article thereof, as well as an adhesive material or adhesive preparation for a medical material or a hygiene material, which is made of the composition or the product.

### Background Art

Conventionally, sheet materials of various gel compositions such as a hydrogel composition, an acrylic gel composition and the like have been used as medical materials or hygiene materials. These gel compositions show adhesion to the skin and followability to skin deformation since they have adhesiveness and flexibility.

However, of the above-mentioned adhesive compositions, a hydrogel composition containing water is weak to dryness, tends to lose water with time to become hard and has poor stability. In addition, a hardened gel composition is insufficient in adhesion to the skin and followability to skin deformation.

Incidentally, as a gel composition free of water, JP-A-3-220120 (patent document 1) discloses an acrylic gel composition retaining an organic liquid component by crosslinking an acrylic adhesive.

However, since molding of the above-mentioned acrylic gel compositions into a sheet material having a certain level of thickness is difficult, the ability thereof to retain or absorb a chemical substance such as a drug and the like, an organic liquid component and the like is limited, and the compositions cannot easily retain the aforementioned substance and the like in a large amount. Moreover, it is somewhat difficult to mold the above-mentioned compositions into molded articles with a large thickness. When they are adhered to the skin, a further improvement may be required of the ability to absorb external impacts and the like and protect the skin.

As other gel compositions, a gel composition obtained by crosslinking a synthetic rubber polymer can be used. JP-A-10-151185 (patent document 2) discloses a gel composition containing 100 parts by weight of a rubber component containing a rubber having a functional group and 20-80 parts by weight of a liquid oil compatible with the rubber component, which are crosslinked.

However, the above-mentioned patent document 2 does not disclose a gel composition containing a rubber component containing a rubber having a functional group and not less than 34.6 wt% of a liquid oil relative to the total weight of the composition for gel preparations. In fact, the adhesive tape for skin adhesion of Comparative Example 2 described in the above-mentioned patent document 2 uses a composition for gel preparations, which contains about 34 wt% of an organic liquid oil component. However, its skin adhesiveness was evaluated to be "low", and adhesive residue was evaluated to be "apparent" or "intensive", and a satisfactory effect is not achieved.
patent document 1: JP-A-3-220120
patent document 2: JP-A-10-151185

### Disclosure of the Invention

### Problems to be Solved by the Invention

It is therefore an object of the present invention to provide a composition for a medical material or a hygiene material, which can retain a considerable amount of a chemical substance such as a drug and the like or an organic liquid component, is associated with less limitation on molding, does not easily drop off when adhered to the skin, provides a better feeling during adhesion to the skin, produces decreased adhesive residue when detached from the skin, and shows good followability to the skin.

### Means of Solving the Problems

The present inventors have conducted intensive studies in an attempt to solve the above-mentioned problems and found that a gel composition obtained by crosslinking a composition for gel preparation comprising a liquid rubber having at least 3 crosslinkable functional groups per molecule and 34.6 - 64.6 wt% of an organic liquid component relative to the total weight of the composition for gel preparation and a tackifier can be molded into a shape having a certain level of thickness, can contain a chemical substance such as a drug and the like and an organic liquid component in a large amount per unit area, and improves adhesiveness to the skin and the like, which resulted in the completion of the present invention.

Accordingly, the present invention provides a gel composition for a medical material or a hygiene material obtained by preparing a composition for gel preparation comprising a liquid rubber having at least 3 crosslinkable functional groups per molecule and 34.6 - 64.6 wt% of an organic liquid component relative to the total weight of the composition for gel preparation and a tackifier, and crosslinking the composition.

Moreover, the present invention provides a molded article obtained by molding the above-mentioned gel composition, and an adhesive material or adhesive preparation having the molded article on at least one surface of a support.

The present invention relates to
(1) a gel composition obtained by crosslinking a composition for gel preparation comprising a liquid rubber having at least 3 crosslinkable functional groups per molecule and 34.6 - 64.6 wt% of an organic liquid component relative to the total weight of the composition for gel preparation, and a tackifier,
(2) the gel composition of the above-mentioned (1), wherein the liquid rubber is a liquid isoprene rubber haying at least 3 crosslinkable functional groups per molecule,
(3) the gel composition of the above-mentioned (1) or (2), wherein the liquid rubber is contained in a proportion of 17.4 - 32.2 wt% relative to the total weight of the composition for gel preparation,
(4) the gel composition of any of the above-mentioned (1) to (3), wherein the organic liquid component is one or more kinds selected from the group consisting of a fat or oil, a hydrocarbon, a fatty acid ester and a higher fatty acid,
(5) the gel composition of the above-mentioned (4), wherein the fatty acid ester is a fatty acid monoalkyl ester,
(6) the gel composition of the above-mentioned (5), wherein the fatty acid monoalkyl ester is one or two kinds selected from the group consisting of isopropyl myristate and isopropyl palmitate,
(7) the gel composition of any of the above-mentioned (1) to (6), wherein the tackifier is contained in a proportion of 17.3 - 32.2 wt% relative to the total weight of the composition for get preparation,
(8) a molded article obtained by molding the gel composition of any of the above-mentioned (1) to (7),
(9) an adhesive material comprising the molded article of the above-mentioned (8) formed on at least one surface of a support, and
(10) an adhesive preparation comprising the molded article of the above-mentioned (8) comprising a drug, which is formed on at least one surface of a support.

### Effect of the Invention

The present invention can provide a composition for a medical material or a hygiene material, which can retain a considerable amount of a chemical substance such as a drug and the like or an organic liquid component, is associated with less limitation on molding, does not easily drop off when adhered to the skin, provides a better feeling during adhesion to the skin, produces decreased adhesive residue when detached from the skin, and shows good followability to the skin.

### Best Mode for Carrying out the Invention

The gel composition of the present invention is obtained by crosslinking a composition for gel preparation comprising a liquid rubber having at least 3 crosslinkable functional groups per molecule and 34.6 - 64.6 wt% of an organic liquid component relative to the total weight of the composition for gel preparation, and a tackifier.

In the present invention, the above-mentioned liquid rubber having at least 3 crosslinkable functional groups per molecule forms a network structure upon crosslinking of the composition for gel preparation containing the same, and imparts flexibility and shape retaining property to the gel composition. The liquid rubber is not particularly limited as long as it has at least 3 crosslinkable functional groups in a molecule, and examples thereof include liquid isoprene rubbers having the aforementioned crosslinkable functional groups, liquid butadiene rubbers having the aforementioned crosslinkable functional groups, liquid isobutylene rubbers having the aforementioned crosslinkable functional groups and the like, which may be used alone or in a combination of two or more kinds thereof. The liquid rubber is liquid even though it has a high molecular weight, and therefore, the gel composition can easily contain a large amount of an organic liquid component. Since flexibility can be easily imparted to the gel composition, a liquid isoprene rubber containing the aforementioned functional groups is preferably used. The "liquid" in the present specification means the presence of flowability at 25°C.

While the weight average molecular weight of the liquid rubber having at least 3 crosslinkable functional groups per molecule is not particularly limited as long as the rubber component is a liquid, it is preferably 1,000-60,000, more preferably 10,000-40,000, most preferably 20,000-30,000. When it is less than 1,000, the gel composition cannot easily contain an organic liquid component in a large amount, or the flexibility of the gel composition may decrease. On the other hand, when it exceeds 60,000, the rubber may not be liquid, and the uniformity of the gel composition may not be maintained easily, possibly making it difficult to secure the compatibility of the aforementioned liquid rubber with other components in the gel composition.

The "weight average molecular weight" used herein means the value measured by gel permeation chromatography under the following conditions:
(analysis conditions)
gel filtration chromatography apparatus: HLC8120 (manufactured by Tosoh Corporation)
column: TSKgel GMH-H(S) (manufactured by Tosoh Corporation) standard: polystyrene
eluent: tetrahydrofuran
flow rate: 0.5 ml/min
measurement temperature: 40°C
detection: differential refractometer

In the composition for gel preparation used for the preparation of the gel composition of the present invention, the amount of the liquid rubber having at least 3 crosslinkable functional groups per molecule is not particularly limited. However, it is preferably 17.4 - 32.2 wt%, more preferably 22.4 - 32.2 wt%, relative to the total weight of the composition for gel preparation. When the amount of the aforementioned liquid rubber is lower than 17.4 wt%, sufficient adhesiveness may not be achieved. On the other hand, when it exceeds 32.2 wt%, adhesive residue may be developed or the flexibility may decrease.

When not less than three functional groups are present in a molecule, the aforementioned liquid rubber efficiently forms a network structure and the effect of the present invention is sufficiently expressed. From such viewpoint, the number of the crosslinkable functional groups in a molecule of the aforementioned liquid rubber is not less than 3, preferably not less than 5, more preferably not less than 8, most preferably not less than 10.

In the present invention, since the above-mentioned liquid rubber has not less than three crosslinkable functional groups in a molecule, a three-dimensional network structure can be formed without the necessity to introduce a branch agent and the like into the main chain of the liquid rubber.
As a result, a large amount of an organic liquid component can be contained in the gel composition.

On the other hand, the number of the crosslinkable functional groups in a molecule is preferably not more than 20, more preferably not more than 15, and most preferably not more than 12, since too many numbers of the functional groups in a molecule increases the amount of necessary crosslinking agent. This is because too many numbers of the functional groups in a molecule produces many residual active groups and may influence the components contained in a gel composition such as a drug and the like, as well as decrease the flexibility of the gel composition, and may degrade the followability to the skin deformation.

The number of crosslinkable functional groups in a molecule of the liquid rubber is determined as follows. That is, the total number of the crosslinkable functional groups in the liquid rubber in a sample is determined, which is divided by the number average molecular weight of the liquid rubber to give the number of the crosslinkable functional groups per 1 molecule of the liquid rubber. When the functional group is a carboxyl group, the number of the carboxyl groups in the sample is determined by a general acid number measurement method using potassium hydroxide. The number average molecular weight of the liquid rubber here means a value obtained under the same conditions as for the above-mentioned weight average molecular weight.

The crosslinkable functional groups in the above-mentioned liquid rubber are not particularly limited and, for example, amino group, carboxyl group, hydroxyl group, chlorosulfone group, ester group, epoxy group, isocyanate group, methylol group, sulfonic acid group, mercapto group, and the like can be used. These may be used alone or in a combination of two or more kinds thereof. From the aspect of formation of a crosslink with an epoxy compound as the below-mentioned crosslinking agent, carboxyl group and hydroxyl group are preferable.

In the present invention, the gel composition is crosslinked. While the crosslinking treatment is not particularly limited, for example, it is achieved by a physical crosslinking treatment by way of irradiation of UV light, electron beam and the like or a chemical crosslinking treatment using various crosslinking agents.

To prevent an adverse influence on the component contained in a gel composition, such as a drug and the like, the gel composition is preferably crosslinked by adding a crosslinking agent to the gel composition. Examples of the crosslinking agent include isocyanate compounds such as amine compound, epoxy compound, isocyanate compounds such as trifunctional isocyanate and the like, organic peroxide, organic metal salt, metal alcoholate, metal chelate compound, multifunctional compound (multifunctional external-crosslinking agent, monomers for multifunctional internal-crosslinking such as diacrylate, dimethacrylate and the like) and the like, which may be used alone or in a combination of two or more kinds thereof. Among the aforementioned crosslinking agents, epoxy compound is preferably used. This is because epoxy group that reacted with the carboxyl group in the liquid rubber molecule develops hydroxyl group, and the hydroxyl group is reactive with other carboxyl group, which is advantageous to the formation of a network structure.

While the amount of the above-mentioned crosslinking agent to be blended is not particularly limited as long as a sufficient crosslinking structure can be formed in the gel composition, the ratio of the total number (A) of the functional groups of the crosslinking agent in the composition for gel preparation to the total number (B) of the functional groups in the liquid rubber molecule in the composition ((A/B)×100) [%] is preferably not less than 50 [%], more preferably not less than 80%. While the upper limit of the amount of the crosslinking agent to be blended is not particularly limited, the aforementioned ratio is preferably not more than 300%, and more preferably not more than 200%, to prevent an adverse influence on the component contained in the gel composition, such as a drug and the like.

Whether the gel composition of the present invention is crosslinked is judged by immersing a gel composition sample in toluene at ambient temperature for 6 days, and visually observing the presence or absence of an insoluble component. When the presence of an insoluble component is confirmed, the gel composition can be said to be crosslinked.

In the present invention, the gel composition contains an organic liquid component. The component is combined with the network structure formed by crosslinking of the aforementioned liquid rubber and enhances the flexibility of the gel composition. When a chemical substance such as a drug and the like is contained in the gel composition, the organic liquid component functions to dissolve or disperse same. The organic liquid component usable in the present invention needs to be compatible with the above-mentioned liquid rubber in the gel composition. It is preferably liquid and nonvolatile at ambient temperature. In the present invention, fats and oils such as olive oil, castor oil, lanolin and the like, hydrocarbons such as squalene and liquid paraffin, fatty acid esters such as fatty acid alkyl ester and the like, higher fatty acids such as oleic acid and caprylic acid, and the like are preferable, and they may be used alone or in a combination of two or more kinds thereof.

Particularly, as in the below-mentioned patch preparation, when the gel composition of the present invention contains a drug, fatty acid esters are preferably used to promote transdermal absorption of the drug. Examples of fatty acid esters include fatty acid dialkyl esters such as dioctyl phthalate, diisopropyl adipate, diethyl sebacate and the like, fatty acid monoalkyl esters and the like. To efficiently promote transdermal absorption of the drug, fatty acid monoalkyl esters are preferable.

As regards the fatty acid monoalkyl esters, when a fatty acid constituting same has an unnecessarily large or small number of carbons, the compatibility with the aforementioned liquid rubber may be degraded or the acid may be volatilized during a heating step for the production of a preparation.

In the present invention, therefore, fatty acid monoalkyl esters composed of saturated or unsaturated higher fatty acid having a carbon number of 12-16, more preferably 12-14, and a saturated or unsaturated lower monovalent alcohol having a carbon number of 1-4 are preferably employed. From the aspect of preservation stability, those composed of a saturated higher fatty acid and a saturated lower monovalent alcohol are preferably used, since they are free of oxidative decomposition and the like. As the aforementioned higher fatty acid, lauric acid (C12), myristic acid (C14), palmitic acid (C16) can be mentioned, with particular preference given to myristic acid and palmitic acid. Examples of the lower monovalent alcohol include methyl alcohol, ethyl alcohol, propyl alcohol and butyl alcohol. They are not limited to straight chain alcohols and may be branched alcohols. Particularly, isopropyl alcohol is preferable. Accordingly, the most preferable fatty acid monoalkyl esters usable in the present invention are isopropyl myristate and isopropyl palmitate.

The content of the aforementioned organic liquid component to be contained is within the range of 34.6 - 64.6 wt% relative to the total weight of the composition for gel preparation. When it is less than 34.6 wt%, the gel obtained after crosslinking reaction may become too hard to degrade the skin adhesiveness and followability to the skin, as well as decrease the diffusion transferability of a compounding component such as a drug etc. in the gel composition and absorbability thereof. On the other hand, when the content of the organic liquid component exceeds 64.6 wt%, gel formation may not be available and the composition may not be molded into a given shape.

As mentioned above, the gel composition of the present invention contains an organic liquid component in an amount larger than that of the aforementioned liquid rubber. This may be the reason for the structure wherein the organic liquid component is simply included in the network structure of the liquid rubber formed by crosslinking of the composition. Rather, however, it is assumed that the liquid rubber component is included in the organic liquid component in the structure. As a result, the gel composition of the present invention molded into a given shape and applied to the skin as a molded article is considered to show optimal flexibility permitting easy followability and adhesion to the deformation of the skin.

In the present invention, while the content ratio of the organic liquid component to the above-mentioned liquid rubber in the composition for gel preparation (organic liquid component content/liquid rubber content) is not particularly limited, it is preferably 1.1 - 3.8, more preferably 1.6 - 3.8. When the aforementioned ratio is less than 1.1, the adhesiveness of the obtained gel composition may be too strong to cause skin irritation. When it exceeds 3.8, a sufficient adhesiveness may not be achieved.

For preparation of the gel composition of the present invention, the composition for gel preparation contains a general tackifier. Examples of the tackifier include naturally occurring substances such as rosin resin, terpene resin and the like, and derivatives thereof, aliphatic petroleum resin, alicyclic petroleum resin, synthesis resins such as alicyclic saturated hydrocarbon resin, aromatic petroleum resin, coumarone inden resin, styrene resin, phenol resin, xylene resin and the like, and the like. These tackifiers are preferably contained within the range of 17.3 - 32.2 wt% relative to the total weight of the composition for gel preparation. While the content ratio of the aforementioned tackifier to the above-mentioned liquid rubber in the composition for gel preparation (tackifier content/liquid rubber content) is not particularly limited, it is preferably 0.7 - 2.0. When the aforementioned ratio is less than 0.7, the obtained gel may become too hard to degrade the skin adhesiveness and followability to the skin, and when it exceeds 2.0, an adhesive residue may remain on the skin.

The gel composition of the present invention may contain other optional components as long as the effect of the present invention is not inhibited. For example, antioxidants such as ascorbic acid and the like, anti-aging agents, fillers, colorants, softening agents and the like may be contained.

The gel composition of the present invention can be produced by mixing the above-mentioned liquid rubber and the organic liquid component and tackifier in the presence of a solvent, where necessary, adding a crosslinking agent thereto to give a composition for gel preparation, feeding the composition into a mold having a given shape, evaporating the solvent at a suitable temperature to allow crosslinking of liquid rubber, aging where necessary and the like.

In addition, the gel composition of the present invention can be molded into a molded article having a given shape. Examples of the shape of the molded article include sheet article, plate article, block article and the like. For application to medical materials or hygiene materials, a sheet article, i.e., a sheet material, is preferable.

The above-mentioned molded article can be formed into a sheet material having a certain thickness of, for example, not less than 0.5 mm, not less than 1 mm, not less than 2 mm or not less than 3 mm and not more than 10 mm. Such sheet material has a greater thickness than that of an adhesive layer of conventional patches, and is advantageous in that a large amount of an organic liquid component, a chemical substance such as a drug and the like can be contained therein. When desired, an adhesive may be separately added to strengthen the adhesiveness of the molded article itself. When desired, moreover, a known release liner may be laminated on the surface to protect the surface of the molded article until use.

Specifically, the molded article of the present invention can be used as a matrix of a matrix type adhesive preparation, a pad-shaped reservoir of a reservoir type adhesive preparation and the like. As a hygiene material, moreover, a given shape, for example, a sheet-like molded article (sheet material) can be used as a gauze substitute for an adhesive plaster, a base material of a gel patch, a substitute for nonwoven fabric of a wound covering dressing and the like.

In the present invention, moreover, for use as a medical material or hygiene material, for example, a patch wherein the molded article of the present invention is formed at least on one surface of a support can be produced. Such patch is used for protecting a lesion or wound on the skin and the like. While the support to be used in the present invention is not particularly limited, one substantially impermeable to the components contained in the molded article; in other words, a support through which the components contained in the molded article are less likely to permeate and lost from the back face thereof, is preferable.

Moreover, the above-mentioned patch can be processed into a patch preparation by impregnating the molded article formed on a support with a drug. The drug usable here is preferably one that can be administered to mammals such as human and the like. Examples of the drug specifically include general anesthetics, hypnotic sedatives, antiepileptic drugs, antipyretic analgesic antiphlogistic drugs, anti-vertiginous drugs, psychoneurotic drugs, topical anesthetics, skeleton muscle relaxants, autonomic drugs, anti-convulsants, anti-parkinsonian drugs, anti-histamine drugs, cardiac stimulants, drugs for arrhythmia, diuretics, hypotensive drugs, vasoconstrictors, coronary vasodilators, peripheral vasodilators, arteriosclerosis drugs, drugs for circulatory organ, respiratory stimulants, antitussive expectorants, hormone drugs, external drugs for mattery diseases, analgesic-antipruritic-styptic-antiphlogistic drugs, drugs for parasitic dermatic diseases, drugs for arrest of bleeding, gout treatment drugs, drugs for diabetes, drugs for anti-malignant tumor, antibiotics, chemical therapy drugs, narcotics, quit smoking aids and the like. While the content of the aforementioned drug in a gel composition is not particularly limited as long as it is an effective amount capable of affording the effect of the drug to be transdermally absorbed and does not impair the property of the gel composition of the present invention, it is preferably 0.1-60 wt%.

### Examples

The present invention is explained in detail by referring to Examples.

Using the compositions for gel preparation having the compositions shown in Table 1, molded articles of the gel compositions of Example 1 - Example 3 and molded articles of the gel compositions of Comparative Example 1 and Comparative Example 2 were prepared. Table 1 shows the content ratios of organic liquid component to liquid rubber having at least 3 crosslinkable functional groups per molecule (organic liquid component/liquid rubber), the content ratios of tackifier to organic liquid component (tackifier/organic liquid component) and the content ratios of tackifier to the aforementioned liquid rubber (tackifier/liquid rubber).

**Table 1**

| | Example 1 | Example 2 | Example 3 | Comp. Ex. 1 | Comp. Ex. 2 |
|---|---|---|---|---|---|
| liquid rubber (wt%) | 29.7 | 24.8 | 19.9 | 14.9 | 34.6 |
| organic liquid component (wt%) | 39.5 | 49.5 | 59.5 | 69.7 | 29.5 |
| Tackifier (wt%) | 29.7 | 24.8 | 19.9 | 14.9 | 34.6 |
| crosslinking agent (wt%) | 1.1 | 0.9 | 0.7 | 0.5 | 1.3 |
| total amount (wt%) | 100.0 | 100.0 | 100.0 | 100.0 | 100.0 |
| organic liquid component/liquid rubber | 1.3 | 2.0 | 3.0 | 4.7 | 0.9 |
| tackifier/organic liquid component | 0.8 | 0.5 | 0.3 | 0.2 | 1.2 |
| tackifier/liquid rubber | 1.0 | 1.0 | 1.0 | 1.0 | 1.0 |

In Table 1, the "liquid rubber" is a liquid rubber having at least 3 crosslinkable functional groups per molecule, and used was 10 functional carboxylic liquid polyisoprene (weight average molecular weight 25,000) having 10 functional groups in a molecule. As the "organic liquid component", isopropyl myristate was used, and as the "crosslinking agent", a compound containing 4 functional epoxy groups (number average molecular weight 366) is used, which were blended in amounts that achieved the ratio of total number of the function groups in the crosslinking agent (A) to the total number of the function groups in the liquid rubber molecule (B) in the composition for gel preparation, ((A/B)×100) [%], of 100%. As the "tackifier", an alicyclic saturated hydrocarbon resin (softening point 100°C) was used. The aforementioned respective components were mixed, stirred and poured into a container, and the mixture was heated at 120°C for 3 hr to allow crosslinking reaction and aged at 80°C for 72 hr to give molded articles of the gel compositions of Examples and Comparative Examples. In the visual examination of the Examples and Comparative Examples, each adhesive layer did not lose shape even when the test piece was tilted 90° under an atmosphere of 25°C, based on which formation of gel was assumed.

The molded articles of the gel compositions of Examples and Comparative Examples were evaluated for the adhesiveness, adhesive residue and flexibility. The evaluation method and evaluation criteria of each evaluation item are shown below.

### (1) Adhesiveness

Evaluation method: respective samples of Examples and Comparative Examples were stabilized by being stood in a temperature-controlled room at 23°C, relative humidity 65% for not less than 1 hr. The samples were formed into cylindrical shape of diameter 50 mmxthickness 10 mm using a metal mold and used as test pieces. The test pieces were placed on a horizontal table in this temperature-controlled room, and a probe was pressed against the top surface from above. Used was a 10 g circular probe having a diameter of 12 mm and a smooth bottom surface. The pressed probe was stood for 10 sec and lifted above, and the maximum stress was measured by Autograph AG-IS (manufactured by SHIMADZU Corporation).

Evaluation criteria: maximum stress: × less than 100 (adhesion itself to the skin is difficult), Δ not less than 100 and less than 300 (can be adhered to the skin but easily drops off), O not less than 300 and less than 450 (can be adhered to the skin, hardly drops off, and less feeling of adhesion to the skin), ⊙ not less than 450 and less than 800 (can be adhered to the skin, hardly drops off, and clear feeling of adhesion to the skin), and O not less than 800 (can be adhered to the skin, hardly drops off, strong feeling of adhesion, and slight skin irritation).

### (2) Adhesive residue

Evaluation method: respective samples of Examples and Comparative Examples were formed into cylindrical shape of diameter 50 mmxthickness 10 mm using a metal mold and used as test pieces. The bottom surface of the test pieces was adhered to the skin surface for 30 min, and the skin condition after detachment was evaluated according to the following evaluation criteria.

Evaluation criteria: when detached from the skin: ○ absence of adhesive residue, and × presence of adhesive residue.

### (3) Flexibility

Evaluation method: Respective samples of Examples and Comparative Examples were formed into a cylindrical shape of diameter 50 mmxthickness 10 mm using a metal mold and used as test pieces. The bottom surface of the test pieces were adhered to the bending part or movable portion of the skin, and skin followability was evaluated according to the following evaluation criteria.

Evaluation criteria: ⊙ skin followability is extremely good, ○ skin followability is good, × drop off from the skin without skin followability when impact is applied to the adhesion site.

The evaluation results of the above are shown in Table 2.

**Table 2**

| | adhesiveness | | adhesive residue | flexibility |
|---|---|---|---|---|
| Example 1 | 750 mN | ⊙ | ○ | ⊙ |
| Example 2 | 500 mN | ⊙ | ○ | ⊙ |
| Example 3 | 300 mN | O | ○ | ⊙ |
| Comparative Example 1 | 200 mN | Δ | ○ | ⊙ |
| Comparative Example 2 | 1400 mN | ○ | × | ○ |

As is clear from Table 2, Example 1 and Example 2 obtained from the compositions for gel preparation containing 29.7 wt% and 24.8 wt% of a liquid rubber having at least 3 crosslinkable functional groups per molecule relative to the total weight of the composition for gel preparation received good evaluation as to the adhesiveness, adhesive residue and flexibility. In Example 3 containing a somewhat greater amount of an organic liquid component, the evaluation of adhesiveness decreased a little, but adhesiveness was sufficient to prevent dropping off from the skin. In Example 2 and Example 3 containing a large amount of an organic liquid component, a soft feeling of adhesion was observed as compared to Example 1.

In contrast, in Comparative Example 1, wherein the composition for gel preparation showed a content ratio of the liquid rubber having at least 3 crosslinkable functional groups per molecule and the tackifier of 1:1 as in Example 1, but contained the organic liquid component in a proportion of 69.7 wt% relative to the total weight of the composition for gel preparation, the adhesiveness was insufficient and the composition easily dropped off from the skin. In Comparative Example 2 obtained from a composition for gel preparation wherein the content of the organic liquid component in the composition was 29.5 wt%, which is lower than the range of the content of the component in the present invention, the adhesiveness was too strong to cause skin irritation.

From the above results, it was shown that the molded articles of the gel compositions of Examples of the present invention had adequate adhesiveness and flexibility in a good balance as compared to the molded articles of the gel compositions of Comparative Examples, and are suitable as a patch causing less adhesive residue upon peeling off from the skin.

## Claims

1. A gel composition obtainable by crosslinking a composition for gel preparation comprising a liquid rubber having at least 3 crosslinkable functional groups per molecule and 34.6 - 64.6 wt% of an organic liquid component relative to the total weight of the composition for gel preparation, and a tackifier.

2. The gel composition of claim 1, wherein the liquid rubber is a liquid isoprene rubber having at least 3 crosslinkable functional groups per molecule.

3. The gel composition of claim 1 or 2, wherein the liquid rubber is contained in a proportion of 17.4 - 32.2 wt% relative to the total weight of the composition for gel preparation.

4. The gel composition of any of claims 1 to 3, wherein the organic liquid component is one or more kinds selected from the group consisting of a fat or oil, a hydrocarbon, a fatty acid ester and a higher fatty acid.

5. The gel composition of claim 4, wherein the fatty acid ester is a fatty acid monoalkyl ester.

6. The gel composition of claim 5, wherein the fatty acid monoalkyl ester is one or two kinds selected from the group consisting of isopropyl myristate and isopropyl palmitate.

7. The gel composition of any of claims 1 to 6, wherein the tackifier is contained in a proportion of 17.3 - 32.2 wt% relative to the total weight of the composition for gel preparation.

8. A molded article obtainable by molding the gel composition of any of claims 1 to 7.

9. An adhesive material comprising the molded article of claim 8 formed on at least one surface of a support.

10. An adhesive preparation comprising the molded article of claim 8 further comprising a drug, which is formed on at least one surface of a support.

## Patentansprüche

1. Gelzusammensetzung, erhältlich durch Vernetzen einer Zusammensetzung für die Gelherstellung, umfassend einen flüssigen Kautschuk mit wenigstens 3 vernetzungsfähigen funktionellen Gruppen pro Molekül und 34,6 bis 64,6 Gew.-% einer organischen flüssigen Komponente, bezogen auf das Gesamtgewicht der Zusammensetzung für die Gelherstellung, sowie einen Klebrigmacher.

2. Gelzusammensetzung gemäß Anspruch 1, wobei der flüssige Kautschuk ein flüssiger Isoprenkautschuk mit wenigstens 3 vernetzungsfähigen funktionellen Gruppen pro Molekül ist.

3. Gelzusammensetzung gemäß Anspruch 1 oder 2, wobei der flüssige Kautschuk in einem Anteil von 17,4 bis 32,2 Gew.-% enthalten ist, bezogen auf das Gesamtgewicht der Zusammensetzung für die Gelherstellung.

4. Gelzusammensetzung gemäß einem der Ansprüche 1 bis 3, wobei es sich bei der organischen flüssigen Komponente um eine oder mehrere Arten handelt, die aus der Gruppe ausgewählt sind, die aus einem Fett oder Öl, einem Kohlenwasserstoff, einem Fettsäureester und einer höheren Fettsäure besteht.

5. Gelzusammensetzung gemäß Anspruch 4, wobei der Fettsäureester ein Fettsäuremonoalkylester ist.

6. Gelzusammensetzung gemäß Anspruch 5, wobei es sich bei dem Fettsäuremonoalkylester um eine oder zwei Arten handelt, die aus der Gruppe ausgewählt sind, die aus Isopropylmyristat und Isopropylpalmitat besteht.

7. Gelzusammensetzung gemäß einem der Ansprüche 1 bis 6, wobei der Klebrigmacher in einem Anteil von 17,3 bis 32,2 Gew.-% enthalten ist, bezogen auf das Gesamtgewicht der Zusammensetzung für die Gelherstellung.

8. Formkörper, erhältlich durch Formen der Gelzusammensetzung gemäß einem der Ansprüche 1 bis 7.

9. Klebematerial, das den Formkörper gemäß Anspruch 8 umfasst, der auf wenigstens einer Fläche eines Trägers gebildet ist.

10. Klebepräparat, das den Formkörper gemäß Anspruch 8 umfasst, der weiterhin einen Wirkstoff umfasst und auf wenigstens einer Fläche eines Trägers gebildet ist.

## Revendications

1. Composition de gel pouvant être obtenue par réticulation d'une composition pour la préparation d'un gel comprenant un caoutchouc liquide comprenant au moins trois groupes fonctionnels réticulables par molécule et de 34,6 à 64,6 % en poids d'un composant liquide organique par rapport au poids total de la composition pour la préparation d'un gel, et un agent poisseux.

2. Composition de gel selon la revendication 1, dans laquelle le caoutchouc liquide est un caoutchouc isoprène liquide comprenant au moins trois groupes fonctionnels réticulables par molécule.

3. Composition de gel selon la revendication 1 ou 2, dans laquelle le caoutchouc liquide est contenu en une proportion de 17,4 à 32,2 % en poids par rapport au poids total de la composition pour la préparation d'un gel.

4. Composition de gel selon l'une quelconque des revendications 1 à 3, dans laquelle le composant liquide organique est d'un ou plusieurs types choisis dans le groupe constitué par une graisse ou une huile, un hydrocarbure, un ester d'acide gras et un acide gras supérieur.

5. Composition de gel selon la revendication 4, dans laquelle l'ester d'acide gras est un ester monoalkylique d'acide gras.

6. Composition de gel selon la revendication 5, dans laquelle l'ester monoalkylique d'acide gras est d'un ou deux types choisis dans le groupe constitué par le myristate d'isopropyle et le palmitate d'isopropyle.

7. Composition de gel selon l'une quelconque des revendications 1 à 6, dans laquelle l'agent poisseux est contenu en une proportion de 17,3 à 32,2 % en poids par rapport au poids total de la composition pour la préparation d'un gel.

8. Article moulé pouvant être obtenu par moulage de la composition de gel selon l'une quelconque des revendications 1 à 7.

9. Matériau adhésif comprenant l'article moulé selon la revendication 8 formé sur au moins une surface d'un support.

10. Préparation adhésive comprenant l'article moulé selon la revendication 8 comprenant en outre un médicament, qui est formé sur au moins une surface d'un support.
